# EUROPEAN PATENT SPECIFICATION

(11) **EP 1 102 837 B1**
(45) Date of publication and mention of the grant of the patent: **26.05.2010**
(21) Application number: 99931021.2
(22) Date of filing: 02.07.1999
(51) Int. Cl.: C12N 1/20, A23C 19/032

(54) **METHOD OF PREVENTING BACTERIOPHAGE INFECTION OF BACTERIAL CULTURES**
VERFAHREN ZUR VERHINDERUNG VON BAKTERIOPHAGENINFEKTION IN BAKTERIENKULTUREN
PROCEDE DE PREVENTION D'UNE INFECTION DE CULTURES BACTERIENNES PAR DES BACTERIOPHAGES

(30) Priority: 03.07.1998 DK 87898; 06.07.1998 US 91735 P
(43) Date of publication of application: 30.05.2001
(73) Proprietor: Chr. Hansen A/S, 2970 Hoersholm (DK)
(72) Inventor: NILSSON, Dan, DK-3060 Espergaerde (DK); JANZEN, Thomas, DK-1964 Frederiksberg (DK)
(86) International application number: PCT/DK1999/000382
(87) International publication number: WO 2000/001799

(56) References cited:
- WO-A1-98/10089
- US-A- 4 900 669
- RICHARDSON G H ET AL: "Proteinase negative variants of Streptococcus cremoris for cheese starters" JOURNAL OF DAIRY SCIENCE, vol. 66, 1983, pages 2278-2286, XP002082743 cited in the application
- RICHARDSON G H ET AL: "Paired and single strain protease negative lactic Streptococci for cheese manufacture." JOURNAL OF DAIRY SCIENCE, vol. 67, no. 3, 1984, pages 518-521, XP002082744 cited in the application
- NILSSON D & LAURIDSEN A A: "Isolation of purine auxotrophic mutants of Lactococcus lactis and characterization of the gene hpt encoding hypoxanthine guanine phosphoribosyltransferase." MOLECULAR AND GENERAL GENETICS, vol. 235, 1992, pages 359-364, XP002032525 cited in the application
- SANDS J A & AUPERIN D: "Effects of temperature and host cell genetic characteristics on the replication of the lipid-containing bacteriophage PR4 in Escherichia coli." JOURNAL OF VIROLOGY, vol. 22, no. 2, 1977, pages 315-320, XP002082746
- DATABASE WPI Section Ch, Week 8934 Derwent Publications Ltd., London, GB; Class B04, AN 89-246864 XP002082747 & SU 1 439 121 A (AS UKR MOLECULAR BIOL), 23 November 1988 (1988-11-23)

## Description

### FIELD OF INVENTION

The present invention relates to the field of bacterial cultures which are used industrially in the manufacturing of e.g. food products or useful metabolite products. In particular there is provided modified bacterial strains, which, when cultivated in a selected substrate material, are not susceptible to attack by bacteriophages, and have retained their capability of being metabolically active.

### TECHNICAL BACKGROUND AND PRIOR ART

Production failures of bacterial cultures caused by bacteriophage infection are considered to be one of the major problems in industrial use of bacterial cultures. Bacteriophages have been found for many of the bacterial strains used in the industry, such as species of lactic acid bacteria e.g. *Lactococcus* spp., *Lactobacillus* spp., *Leuconostoc* spp., *Pediococcus* spp., and *Streptococcus* spp., *Propionibacterium* spp., *Bifidobacterium* spp, *Staphylococcus* spp. or *Micrococcus* spp. Furthermore, bacteriophage infections are also well known in other industrially useful species such as *Bacillus* spp., *Enterobacteriaceae* spp. including *E. coli, Corynebacterium* spp. *Actinomycetes* spp. and *Brevibacterium* spp.

In the food industry lactic acid bacterial starter cultures are widely used for food fermentations. It appears that among members of the lactic acid bacteria *Lactococcus* spp. are most devastated by bacteriophage infections. A factor, which leads to frequent bacteriophage infections in lactic acid bacterial starter cultures is the fact that the fermentation conditions in the food industry including the dairy industry are generally non-sterile. Thus, it has not yet been possible to eliminate bacteriophage contamination under these industrial conditions.

The lytic development of phages involves adsorption of the phages to the host cell surface, injection of phage DNA into the cell, synthesis of phage proteins, replication of phage DNA, assembly of progeny phages and release of progeny from the host. Cell-mediated mechanisms of interference with any of these events can prevent a phage infection. The ability of bacterial cultures to resist bacteriophage infection during industrial use depends to a large extent on host strain characteristics affecting one or more of the above mechanisms.

Thus, it has been shown that natural bacteriophage resistance or defense mechanisms exist in bacterial strains which ensure a certain level of protection against bacteriophage attack. These natural defense mechanisms include phage adsorption inhibition, prevention of phage DNA penetration, restriction of phage DNA and abortive infection.

The prevention of productive contact between phages and bacterial cells due to altered cell surface receptors for phages greatly reduces the ability of the phages to attack the cells. Adsorption of the phages to the cell surface is not always sufficient for the translocation of the phage DNA. It has been shown that host specific cell membrane proteins are involved in the prevention of phage DNA penetration.

Restriction/modification is a mechanism that operates by the cooperation of two enzyme systems, a DNA-cleaving restriction enzyme and a DNA-modifying enzyme, usually a methylase. The mechanism functions by cleaving the phage DNA, as it enters the cell.

Abortive phage infection is described as a mechanism that interferes with the phage development after phage expression has begun. This may eventually lead to a reduced level or termination of the production of viable phage progeny.

However, like many other traits of bacterial strains which are important for industrial performance, the above described natural phage defense mechanisms have been shown to be unstable characteristics, as they may be mediated by plasmids. Furthermore, these defense mechanisms are often phage specific, i.e. they are only active against a limited range of bacteriophage types. Accordingly, the prior art is not aware of a general and stably maintained host cell associated resistance mechanism against bacteriophage infection.

Based on the above natural defense mechanisms, the industry has designed and implemented strategies to possibly reduce bacteriophage infection of bacterial cultures including starter cultures for the fermentation of dairy products. Currently used strategies include the use of mixed starter cultures and alternate use of strains having different phage susceptibility profiles (strain rotation).

Traditionally, starter cultures in the dairy industry are mixtures of lactic acid bacterial strains. The complex composition of mixed starter cultures ensures that a certain level of resistance to phage attack is present. However, repeated subculturing of mixed strain cultures leads to unpredictable changes in the distribution of individual strains and eventually undesired strain dominance. This in turn may lead to increased susceptibility to phage attack and risk of fermentation failures.

Rotation of selected bacterial strains which are sensitive to different phages is another approach to limit phage development. However, it is difficult and cumbersome to identify and select a sufficient number of strains having different phage type profiles to provide an efficient and reliable rotation program. In addition, the continuous use of strains requires careful monitoring for new infectious phages and the need to quickly substitute a strain which is infected by the new bacteriophage by a phage resistant strain. In manufacturing plants where large quantities of bulk starter cultures are made ahead of time, such a quick response is usually not possible.

Studies have shown that a reduced growth capacity of a bacterial culture such as a proteinase-deficient lactic acid bacterium results in reduced phage proliferation (Richardson et al., 1983, 1984). However, such bacterial strains are still growing and are thus still susceptible to attack by phages.

Thus, the industry is not in the possession of any reliable strategy to secure that bacterial cultures used for industrial manufacturing of food products or other products are resistant against attack by bacteriophages. Furthermore, none of the currently used strategies prevent infections by any type of bacteriophages and none of these strategies are capable of precluding that bacteriophages, by a mutational event, circumvent the resistance mechanisms of the bacterial culture strains.

It is therefore a significant objective of the present invention to provide a method of preventing bacteriophage infection of bacterial cultures which are used in the manufacturing of food products and other products, wherein the cultures are completely resistant to attack by all types of bacteriophages.

The article (Meagher et al, Cell, 1997, vol. 10, pages 521-536) describes protein expression in E. coli minicells by recombinant plasmids.

The article (Helling et al, J.Bacteriol., 1970, vol. 104, no. 2, pages 1027-1029) relates to nalidixic acid-resistant auxotrophs of E. coli.

The article (Worland et al, Australian Journal of Plant Physiology, 1999, 26(6), pages 511-519) describes that Rhizobium purine auxotrophs, perturbed in nodulation, have multiple changes in protein synthesis.

The article (Anton et al, J. Bacteriol 77(1): 117-8, 1959) describes a thymine "requiring" LAB variant. It is not said anywhere in this article that this variant is not DNA replicating. Further, there is not described anything about phage resistance and use for making a food/feed product.

The article (Ross et al, Appl Environ Microbiol, 56(7): 2164-9, 1990) relates to use of *thyA* as a genetic marker. There is not described anything about phage resistance and use for making a food/feed product.

The article (Pedersen et al, Appl Environ Microbiol, 68(6):3010-23, 2002) was published in 2002.

The article (Deutch et al, J Bacteriol 106:197-203, 1971) describes things that may happen during incubating of a thymine auxotroph E. coli.

The article (Breheny et al, Aust J Diary Technol, 30:145-148, 1975) describes lactic acid bacteria (LAB) "locked in protein synthesis". Accordingly, in this article is not disclosed LAB that are not DNA replicating. Further, there is not described anything about phage resistance.

The article (Desjardins et al, J Dairy Sci 73:1478-1484, 1990) describes some kinetic fermentation profiles of a natural wild-type LAB. Since it is wild-type it is self-evident that article does not disclose LAB that are not DNA replicating. Further, there is not described anything about phage resistance.

WO95/10621 (Chr. Hansen) relates to lactic acid bacterial suppressor mutants and their use as selective markers.

### SUMMARY OF THE INVENTION

Accordingly, the invention provides in a first aspect a method of modifying a substrate material by means of a bacterial culture which is capable of being metabolically active in said substrate, whereby the bacterial culture is not susceptible to attack by bacteriophages, the method comprising
(i) isolating a bacterial strain which is not capable of DNA replication, RNA transcription or protein synthesis in said substrate material but is capable of metabolically modifying the substrate material,
(ii) propagating the selected strain in a medium wherein the strain is capable of replicating to obtain a culture of said strain,
(iii) adding the thus obtained bacterial culture to the substrate material and keeping the material under conditions where the culture is metabolically active,
whereby, if the substrate material is contaminated with a bacteriophage, the metabolic activity of the bacterial culture is substantially unaffected by the bacteriophage, and
wherein the bacterial culture is selected from the group consisting of *Lactococcus* spp., *Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Corynebacterium* spp. and *Brevibacterium* spp; and
wherein the substrate material is a starting material for an edible product, the material is selected from the group consisting of milk, a vegetable material, a meat product, a must, a fruit juice, a wine, a dough and a batter; and
wherein the bacterial culture as obtained in the method is used as a starter culture in the preparation of a product selected from the group consisting of a dairy flavour, a product for cheese flavouring, a food product and a feed product.

The invention pertains in another aspect to the use of a culture as obtained in the method of any of claims 1 to 11 as a starter culture in the preparation of a product selected from the group consisting of a dairy flavour, a product for cheese flavouring, a food product and a feed product.

In a further aspect there is provided a mutant lactic acid bacterium, wherein the mutant strain is the thy A mutant Lactococcus lactis strain MBP71 deposited under accession number DSM 12891.

### DETAILED DISCLOSURE OF THE INVENTION

Thus, in its broadest aspect the invention provides a method of modifying a substrate material by means of a bacterial culture which is capable of being metabolically active in said substrate, whereby the bacterial culture is not susceptible to attack by bacteriophages, said method comprising the steps (i) to (iii) as mentioned above. As used herein, the expression "modifying a substrate material" is used interchangeably with the term "fermentation" and relates to any aerobic or anaerobic breakdown of organic compounds by a bacterial culture with the production of an end product. In addition, it will be appreciated that the expression "metabolically active" refers to the capability of the bacterial culture to convert a substrate material such as e.g. milk or a sugar.

In the present context, the expression "not susceptible to attack by bacteriophages" includes the capability of a host cell to be metabolically active even though a bacteriophage adsorbs to the host cell surface and injects its DNA into the host cell. As used herein, the term "bacteriophages" referes to any kind of virus that infects bacteria, including the group of prolate headed bacteriophages, isometric headed bacteriophages and group P335 of bacteriophages.

In accordance with the invention, the method comprises in one aspect the isolation of a bacterial strain which is not capable of DNA replication, RNA transcription or protein synthesis in a specifically defined substrate material, but is capable of metabolically modifying said material. It will be understood that in this context the expression "a specifically defined substrate material" referes to substrate material which is limited with respect to at least one nutrient compound that is required by the bacterial strain for DNA replication, RNA transcription or protein synthesis. Evidently, it was a very surprising finding that it is possible to provide such non-proliferating bacterial strains which are unable to grow in specifically defined substrate materials, but which have retained their capability of being metabolically active. As used herein, the expression "non-proliferating bacterial strain" relates to a bacterial strain which is incapable of multiplying in a specifically defined substrate material.

In a particularly useful embodiment of the present invention the above specific substrate material is limited with respect to at least one nutrient compound that is required by the bacterial strain for DNA replication, RNA transcription or protein synthesis. Such compounds include amino acids or nitrogenous bases such as purine and pyrimidine bases.

Thus, the growth of the bacterial strain is prevented due to the lack of capability of the strains to synthesize the specific compound with respect to which the substrate is limited. Such a mutant strain which has lost the capability of *de novo* synthesising such essentially compounds is also referred to in the art as an "auxotrophic strain". Therefore, in preferred embodiments, the bacterial strain is a mutant strain being auxothrophic in respect of a compound which is not present in the substrate material and which is required by the strain for DNA replication, RNA transcription or protein synthesis.

The substrate material used in the method of the invention may in a further useful embodiment contain at least one compound that inhibits the DNA replication, RNA transcription or the protein synthesis of the bacterial strain. Examples of such compounds include chloramphenicol and erythromycin which affect the ribosomes of the bacterial cell and thus inhibit protein synthesis.

In accordance with the invention, the propagation of the selected strain prior to its use in the present method requires a medium wherein the strain is capable of replicating to obtain a culture of said strain. It is assumed that a medium containing the specific compound which the mutant is unable to synthesize, will restore the capability of the mutant to grow, i.e. capability of DNA replication, RNA transcription and/or protein synthesis.

In a further step of the method according to the invention, the above obtained bacterial culture is added to the above substrate material and kept under conditions where the culture is metabolically active. It will be understood that in this context, the term "conditions" includes the temperature, pH, appropriate composition of the substrate material or presence/absence of an inducer substance, at which the metabolic activity of the bacterial culture is optimal.

Bacteriophages require hosts with intact DNA replication, RNA transcription and protein synthesis in order to become proliferated. Accordingly, bacterial cultures used in the method of the invention are incapable of performing one of the above activities, which makes such bacterial cultures substantially completely resistant to attack by bacteriophages. Thus, the metabolic activity of the bacterial culture is substantially unaffected even if the substrate material is contaminated with bacteriophages. As used herein, the expression "substantially unaffected" indicates that by using conventional detection methods no changes or only minor changes in the metabolic activity can be detected.

It will be appreciated that such auxotrophic bacteria can be provided by subjecting a wild type bacterial strain that, under appropriate conditions, is capable of growing in a substrate material with or without a specific compound needed for DNA replication, RNA transcription or protein synthesis to a mutagenization treatment and selecting a strain that is substantially incapable of growing in the absence of said specific compound.

Suitable mutagens include conventional chemical mutagens and UV light. Thus, as examples, a chemical mutagen can be selected from (i) a mutagen that associates with or becomes incorporated into DNA such as a base analogue, e.g. 2-aminopuririe or an interchelating agent such as ICR-19.1, (ii) a mutagen that reacts with the DNA including alkylating agents such as nitrosoguanidine or hydroxylamine, or ethane methyl sulphonate (EMS).

As an alternative, auxotrophic bacteria can be provided by selecting spontaneously occurring mutants which, compared to the parent strain, has a growth requirement for a compound needed for DNA replication, RNA transcription or protein synthesis.

It will be understood that it is also possible to provide an auxotrophic mutant by site-directed mutagenesis, e.g. by using recombinant DNA techniques, such as gene knock-out techniques, by which the specific gene is disrupted and rendered non-functional in the bacterium. It is also possible to construct the mutated bacterial strains according to the method of the present invention by techniques which involve the loss of part of the chromosome or a nucleotide base or bases in the DNA sequence which renders the specific gene non-functional in the bacterium. An illustrative example of such a deletion strategy is described in details in the below

### Examples.

The above bacterial wild type parent strain can be selected from any industrially suitable bacterial species, i.e. the strain can be selected from the group consisting of *Lactococcus* spp. including *L. lactis, Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Enterobacteriaceae* spp. including *E. coli, Corynebacterium* spp. and *Brevibacterium* spp.

In one specific embodiment of the method according to the invention, a Pur mutant, including the *Lactococcus lactis* strain DN105 (DSM 12289), is used. This acidifying bacterial strain DN105 is a purine auxotrophic mutant capable of acidifying milk, even though the strain is not capable of growth because of its requirement for purine, which is not present in milk in sufficient amounts to support growth of such a bacterium. As it is shown in the below Example 1, strain DN105 is capable of acidifying milk under purine starvation conditions even in the presence of a high concentration of bacteriophages.

In a further useful embodiment of the method according to the invention, a *thyA* mutant, including the *Lactococcus lactis* strain MBP71 (DSM12891), is used. This acidifying bacterial strain MBP71 is a thymidine auxotrophic mutant, which is capable of acidifying milk, even though the strain is not capable of growth, due to its requirement for thymidine, which is not present in milk in sufficient amounts to support growth of such a mutant. As shown in Example 2 below, strain MBP71 is capable of acidifying milk under thymidine starvation conditions even in the presence of a high concentration of bacteriophages.

It is convenient to provide the above bacterial strains both when used as a food or feed production strain and as a production strain for metabolites, as a composition comprising the bacterial strain selected for the specific use. Typically, such compositions contain the bacterium in concentrated form e.g. at a concentration of viable cells (colony forming units, CFUs) which is in the range of 10⁵ to 10¹³ per g of the composition such as in the range of 10⁶ to 10¹² per g. Additionally, the culture composition may contain further components such as bacterial nutrients, cryoprotectants or other substances enhancing the viability of the bacterial active ingredient during storage. The composition can e.g. be in the form of a liquid, frozen or a freeze-dried composition.

As mentioned above, one characteristic of the bacterial culture as used in the method of the invention, is that it is capable of metabolically modifying a specifically defined substrate material even though the strain is incapable of growth in such substrate. When a lactic acid bacterium is used in the method of the invention, the obtained bacterial culture typically has an acidification rate in milk which is at least 10% of that of said culture when it is present in a substrate material where it is capable of DNA replication, RNA transcription and/or protein synthesis. In preferred embodiments, the bacterial strain has an acidification rate in milk which is at least 1 % including at least 5%, such as at least 10%, e.g. at least 15%, such as at least 20%, including at least 25% of that of the culture when it is present in a substrate material where it is capable of DNA replication, RNA transcription and/or protein synthesis.

Typically, the bacterial strain used in the method of the invention is added to the substrate material at a concentration in the range of 10⁵ to 10⁹ CFU/ml or g of the material, such as at least 10⁵ CFU/ml or g of the material, including at least 10⁶ CFU/ml or g of the material, such as at least 10⁷ CFU/ml or g of the material, e.g. at least 10⁸ CFU/ml or g of the material, including at least 10⁹ CFU/ml or g of the material.

Thus, the obtainment of a bacterial culture which is completely resistant to bacteriophage attack according to the method of the invention, can be utilized in all industrial contexts where proliferation of the culture in the substrate material is not a requirement. Dairy fermentations of milk is such an example of an industrial manufacturing process, as proliferation of the lactic acid bacteria during milk fermentation is not a requirement, if the desired taste and acidification of the fermentation product are obtained. Therefore, in useful embodiments, the substrate material is a starting material for an edible product including milk, a vegetable material, a meat product, a must, a fruit juice, a wine, a dough and a batter.

In further embodiments, the substrate material is a starting material for an animal feed such as silage e.g. grass, cereal material, peas, alfalfa or sugar-beet leaf, where bacterial cultures are inoculated in the feed crop to be ensiled in order to obtain a preservation hereof, or in protein rich animal waste products such as slaughtering offal and fish offal, also with the aims of preserving this offal for animal feeding purposes.

Yet another significant application of the method according to the present invention is the use of the bacterial cultures as so-called probiotics. By the term "probiotic" is in the present context understood a microbial culture which, when ingested in the form of viable cells by humans or animals, confers an improved health condition, e.g. by suppressing harmful microorganisms in the gastrointestinal tract, by enhancing the immune system or by contributing to the digestion of nutrients.

It is, as mentioned above, an important objective of the present invention to provide a method of preventing bacteriophage infection of bacterial cultures which are metabolically active by using non-proliferating bacterial cells. In order to be of industrial interest, such metabolic activity should result in the production of a substantial amount of the desired end product. Thus, one possibility of increasing such production by use of a non-proliferating cell is an enhancement of the flux through metabolic pathways.

Accordingly, in one useful embodiment, the bacterial culture used in the method of the invention comprises a genetically modified strain which, relative to its parent strain, is enhanced in at least one metabolic pathway. Such enhanced metabolic activity can e.g. be obtained through an enhanced glycolytic pathway and/or an enhanced flux through the pentose phosphate pathway.

One approach for stimulating the flux through the glycolytic pathway is by increasing the expression of ATPase activity, i.e. an enhanced conversion of ATP to ADP, as described in WO 98/10089. Thus, in one useful embodiment of the invention, the genetically modified strain has, relative to its parent strain, an enhanced ATPase activity.

In one interesting embodiment of the present invention, the genetically modified strain is one wherein the gene coding for an ATPase is under the control of a regulatable promoter. As used herein, the term "regulatable promoter" is used to describe a promoter sequence possibly including regulatory sequences for the promoter, which promoter is regulatable by one or more factors present in the environment of the strain. Such factors include the pH of the growth medium, the growth temperature, a temperature shift eliciting the expression of heat shock genes, the composition of the growth medium including the ionic strength/NaCl content and the growth phase/growth rate of the bacterium. Such a regulatable promoter may be the native promoter or it may be an inserted promoter not naturally related to the gene either isolated from the same bacterial species or it may be a heterologous promoter sequence, i.e. a sequence derived from different bacterial species.

Cells such as "resting cells" or "non-dividing cells" represent other types of non-proliferating cells which are useful in the method of the invention and wherein the above enhancement of the flux through the metabolic pathways is useful. Such cells are incapable of mitosis or meiosis e.g. due to the deficiency of DNA, RNA and/or protein needed for the separation of the cell. Thus, in a particularly useful embodiment, the bacterial culture is one which comprises a bacterial strain which is capable of increasing the size of the cells without mitosis.

In addition, the invention encompasses non-proliferating strains which under specific conditions are incapable of growth. Thus, in an interesting embodiment, the bacterial culture comprises a strain which is a conditional mutant, i.e. a mutant which under predetermined conditions does not perform at least one activity selected from the group consisting of DNA replication, RNA transcription and protein synthesis. Such predetermined conditions include pH, temperature, composition of the substrate material and presence/absence of inducer substances.

One possible means of providing such conditional mutants which are temperature-sensitive is by subjecting a bacterial strain that under appropriate conditions is capable of growing in a substrate material e.g. at a temperature below 30°C to a mutagenization treatment and selecting a mutant strain that is substantially incapable of growth at temperatures below 30°C, but is capable of growth at higher temperatures.

In a further aspect of the invention there is provided a modified lactic acid bacterium that is modified to become incapable of performing DNA replication, RNA transcription or protein synthesis in a specifically defined substrate material which is limited with respect to at least one compound that is required by the bacterial strain for DNA replication, RNA transcription or protein synthesis, said modified bacterial strain is capable of being metabolically active in said substrate material, whereby the strain is not susceptible to attack by bacteriophages, subject to the limitation, that the lactic acid bacterium is not strain DN105 (DSM 12289).

In useful embodiments, the lactic acid bacterium according to the invention is a mutant strain being auxothrophic in respect of a compound which is not present in the substrate material and which is required by the strain for replication.

Presently preferred lactic acid bacteria according to the invention are mutant strains which are *thyA* mutants including *Lactococcus lactis* strain MBP71 deposited under the accession number DSM 12891.

The modified lactic acid bacterium according to the invention is useful as a starter culture in the production of food and feed products. Accordingly, in a further important aspect the invention relates to a starter culture composition comprising the lactic acid bacterium according to the invention.

As it is normal in the production of lactic acid bacterial fermentation processes to apply mixed cultures lactic acid bacteria, a composition will in certain embodiments comprise a multiplicity of strains either belonging to the same species or belonging to different species. Accordingly, in a further important aspect, the invention relates to a starter culture composition comprising a lactic acid bacterium obtainable by the method according to the invention in combination with at least one further lactic acid bacterium. A typical example of such a useful combination of lactic acid bacteria in a starter culture composition is a mixture of the bacterium obtainable by the method according to the invention and one or more *Lactococcus* spp. such as *Lactococcus lactis* subsp. *lactis* or *Lactococcus lactis* subsp. *lactis* biovar. *diacetylactis* or *Leuconostoc* spp. Such a mixed culture can be used in the manufacturing of fermented milk products such as buttermilk and cheese.

In one embodiment, the composition according to the invention, is one which further comprising at least on component enhancing the viability of the bacterial active ingredient during storage including a bacterial nutrient or a cryoprotectant.

It is also an objective of the present invention to provide a method of manufacturing a food or feed product based on the use of the modified bacterium of the invention. Thus, in its broadest aspect, such a method comprises adding a starter culture composition according to the invention to a food or feed product starting material and keeping the thus inoculated starting material under conditions where the lactic acid bacterium is metabolically active. In a particularly useful embodiment, the food product starting material is milk.

In further aspects, the invention relates to the use of a culture as obtained in the method according to the invention as a starter culture in the preparation of a product selected from the group consisting of a dairy flavour, a product for cheese flavouring, a food product and a feed product.

The invention will now be described in further details in the following non-limiting examples and the drawings wherein
Fig. 1. shows the acidification of reconstituted skim milk (RSM) by strain DN105. The pH was followed over time in RSM cultures containing strain *Lactococcus lactis* DN105 inoculated at 1%, 10%, 25% and 50% vol/vol;
Fig. 2 illustrates the development of pH in RSM when inoculated with 25% vol/vol of *Lactococcus lactis* strain CHCC373 or *Lactococcus lactis* strain DN105, in the presence of the bacteriophage strain 836 at a concentration of at least 10⁸ PFU/ml;
Fig. 3A-3H illustrate the development of pH in RSM when inoculated with 25% vol/vol of *Lactococcus lactis* strain DN105 with or without addition of the purine compound hypoxanthine to the culture medium, in the presence of at least 10⁸ PFU/ml of the following bacteriophages: CHPC836 (3A); CHPC412 (3B); CHPC783 (3C); CHPC795 (3D); CHPC710 (3E); CHPC12 (3F); CHPC708 (3G); CHPC814 (3H);
Fig. 4. shows the acidification of RSM by *Lactococcus lactis* strain MBP71 inoculated at different concentrations. The pH of 200 ml RSM cultures comprising strain MBP71 inoculated at 1%, 5%, 10%, 25%, and 50% (v/v) was monitored on-line; and
Fig. 5. shows the acidification of RSM, comprising bacteriophages and/or thymidine, by *Lactococcus lactis* strains MBP71 and CHCC373, which were inoculated at a concentration of 25% (v/v) in a volume of 100 ml RSM. The thymidine concentration was 20 mg/l and bacteriophage CHPC733 was added to a concentration of 2x10⁸ PFU/ml. The pH was monitored by collecting 2.5 ml samples.

### EXAMPLE 1

### The use of a purine auxotrophic Lactococcus lactis strain for obtaining resistance against bacteriophages in milk fermentations

### 1.1. Materials and methods

### (i) Bacterial strains, media and growth conditions

The *Lactococcus lactis* strain DN105 is a purine auxotrophic mutant (Pur) derived from the wild type strain CHCC373 described in Nilsson and Lauridsen (1992). A sample of *Lactococcus lactis* strain DN105 is deposited with Deutsche Sammlung von Mikroorganismen und Zellkulturen GmbH, Mascheroder Weg 1b, D-38124 Braunschweig, Germany on 30 June 1998 under the accession No. DSM 12289.

*Lactococcus lactis* was grown in M17 (Terzaghi & Sandine, 1975) in the chemically defined DN medium (Dickely et al., 1995) or in reconstituted skim milk (RSM) containing 9.5% (w/vol) low fat skim milk powder at 30°C. For the propagation of strain DN105, the medium was supplemented with hypoxanthine to a final concentration of 15-50 mg/I. The Pur phenotype of strain DN 105 was tested by its ability to grow on DN medium with and without hypoxanthine supplement.

### (ii) Bacteriophages and their handling

Bacteriophages were purified by three single plaque isolation steps. The high titer bacteriophage lysates were prepared by consecutive infections of the host strain CHCC373 with the bacteriophage at an MOI (multiplicity of infection) of 0.1 to 1.0. After infection the culture was grown at 30°C in M 17 supplemented with 10 mM CaCl₂ until completed lysis. The lysates were centrifuged for 15 min at 6.000 rpm and the supernatant sterile filtered (0.45 µm, Schleicher und Schuell).

### (iii) Determination of bacteriophage titers

For the determination of bacteriophage titers (plaque forming units per ml) the agar double layer method was used (Adams M.H., 1959; Interscience Publishers, Inc., New York). The bacteriophages used for the acidification test are listed in Table 1 below:

**Table 1. Bacteriophages and phage titers**

| Phage | Titer (PFU/ml) |
|---|---|
| CHPC12 | 4x10¹⁰ |
| CHPC412 | 3x10¹⁰ |
| CHPC708 | 2x10¹⁰ |
| CHPC710 | 2x10¹⁰ |
| CHPC783 | 1x10¹¹ |
| CHPC795 | 7x10¹⁰ |
| CHPC814 | 1x10¹⁰ |
| CHPC836 | 4x10¹⁰ |

### (iv) Test for the acidification of RSM by strain DN105

10-400 ml of an outgrown culture of strain DN105 in M17 was harvested by centrifugation, washed twice with a sterile solution of 0.9% NaCl to remove residual purine compounds and resuspended in 10-400 ml of RSM to give the same cell density as in the outgrown M 17 culture. The resuspended material was used for inoculation of fresh RSM at volume/volume concentrations typically in the range of 10 to 100% (v/v). The pH was monitored either on-line or by measuring the pH of 3 ml samples collected at intervals.

### 1.2. Results of the acidification of RSM by Lactococcus lactis strain DN105

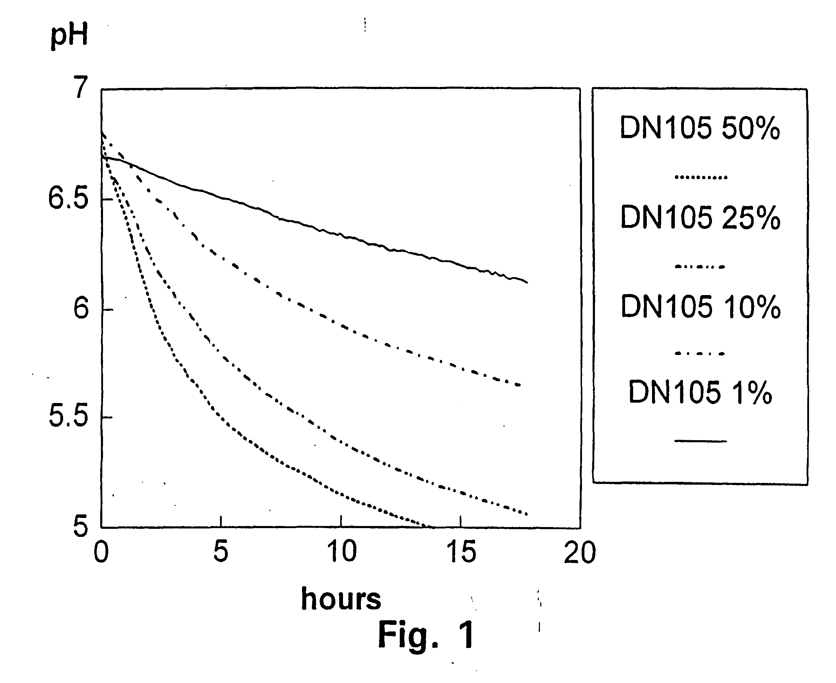

In general, lactic acid bacterial cells which do not have intact DNA replication, RNA transcription and protein synthesis systems are unable to grow and acidify milk. Nilsson and Lauridsen (1992) demonstrated that the purine auxotrophic mutant DN 105 is unable to grow in a medium without purines. It has also been reported that purine auxotrophic mutants of *Lactococcus lactis* not growing in milk are incapable of acidifying such a substrate material (Dickely et al., 1995). To test the ability of strain DN105 to acidify a milk based medium, the strain was inoculated in varying amounts in RSM as described in Materials and Methods and the pH of the substrate material was monitored (Fig. 1).

The results shown in Fig. 1 clearly demonstrates that strain DN 105 was able to acidify milk at least to pH 5.0 even under purine starving conditions.

### 1.3 Studies of the bacteriophage resistance of Lactococcus lactis strain DN105 in RSM

As it is generally known, a bacteriophage requires a susceptible host cell which has intact DNA replication, RNA transcription and protein synthesis systems for development. If a potential host is not capable of performing at least one of these activities, bacteriophages cannot be proliferated in the cell.

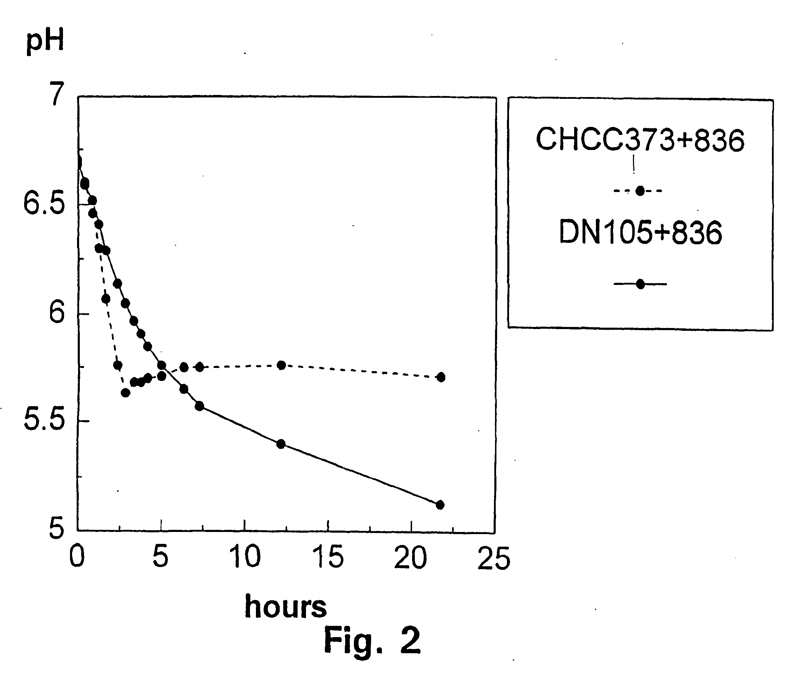

Fig. 2 shows the development of pH in RSM when inoculated with 25% vol/vol of the wild type strain CHCC373 or the mutant strain DN105 in the presence of the bacteriophage CHPC836 at a concentration of at least 10⁸ PFU/ml.

In further experiments the resistance of strain DN105 against various bacteriophages was studied in RSM with and without hypoxanthine added. The bacteriophages were added to RSM at 10⁸ to 10⁹ bacteriophages/ml.

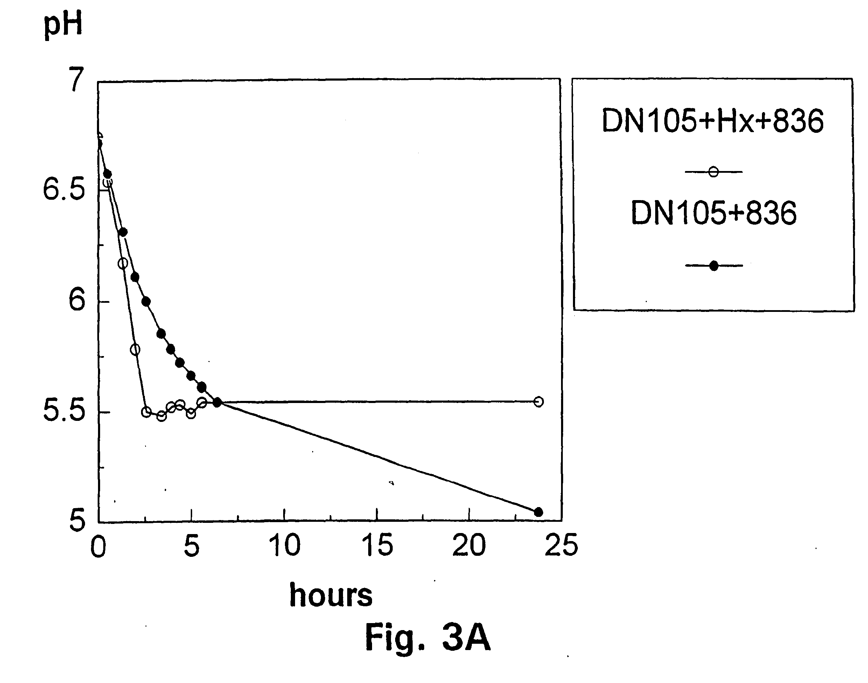

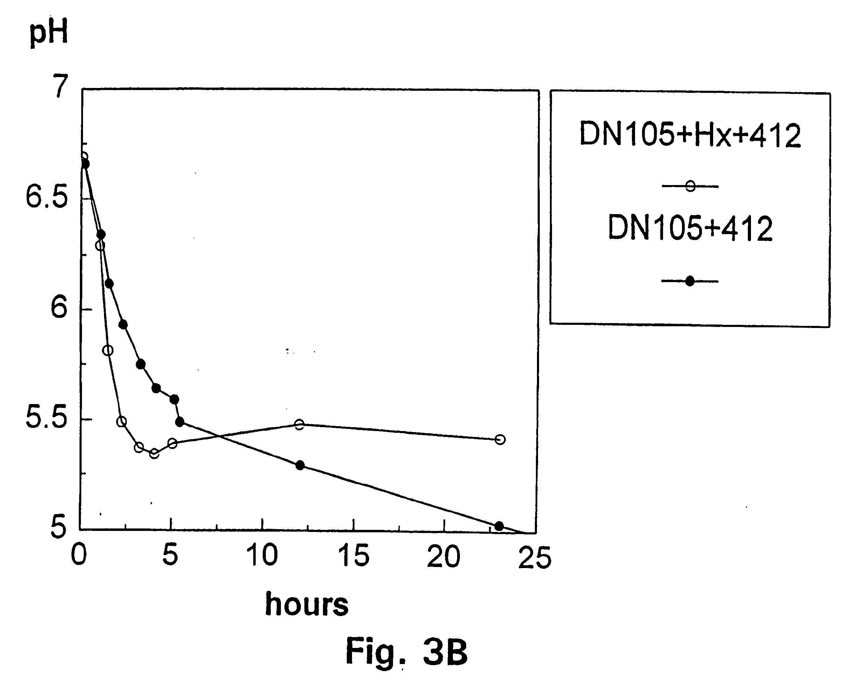

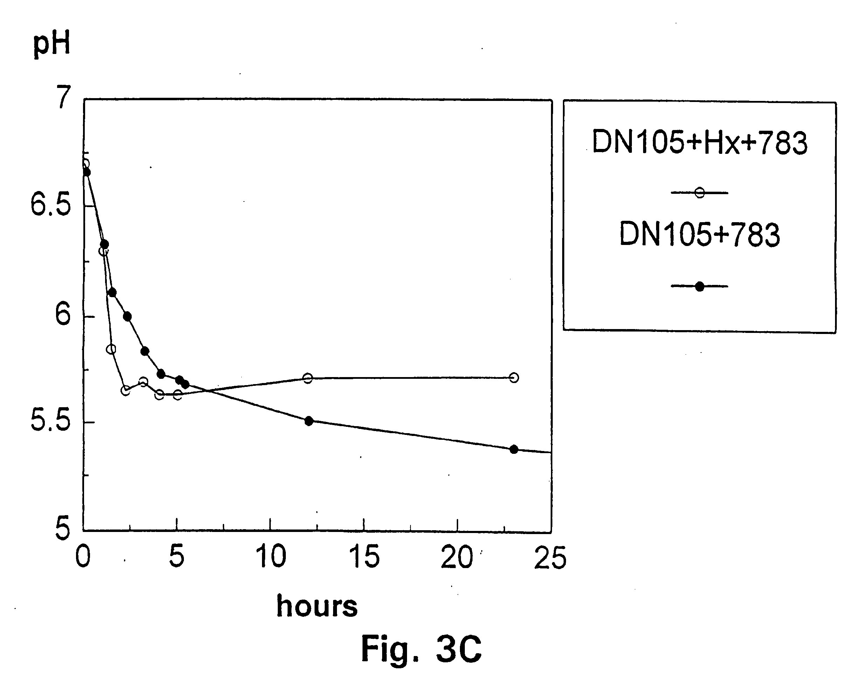

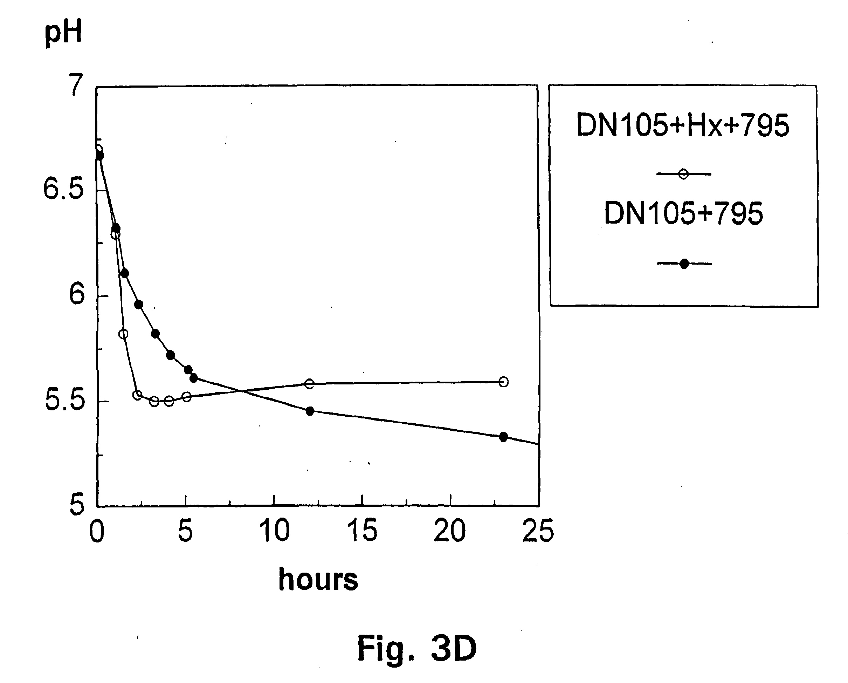

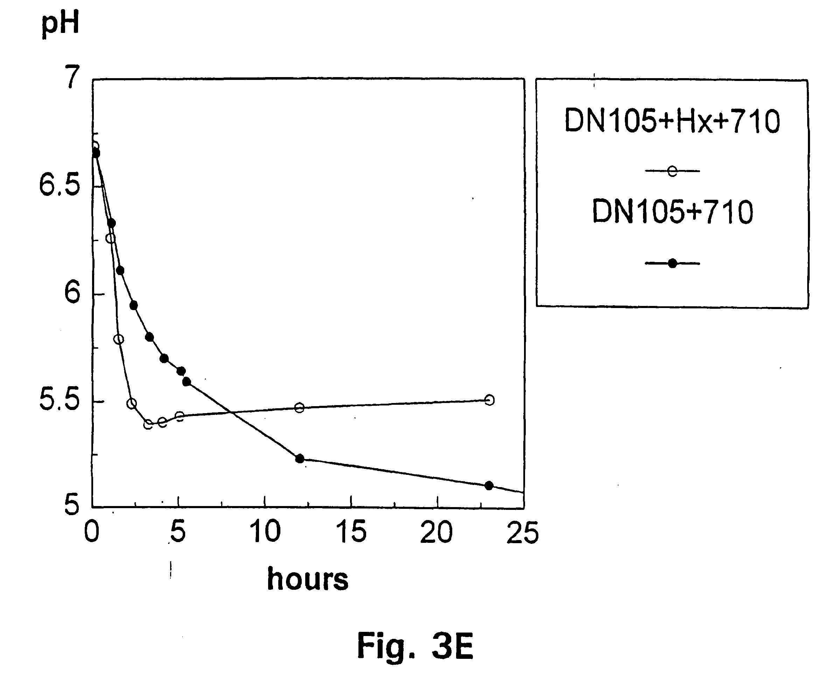

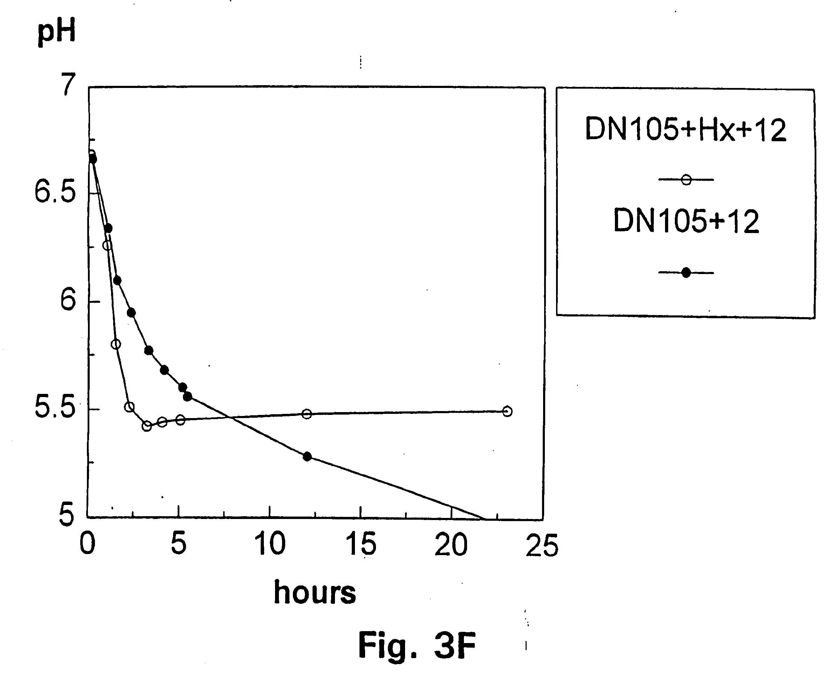

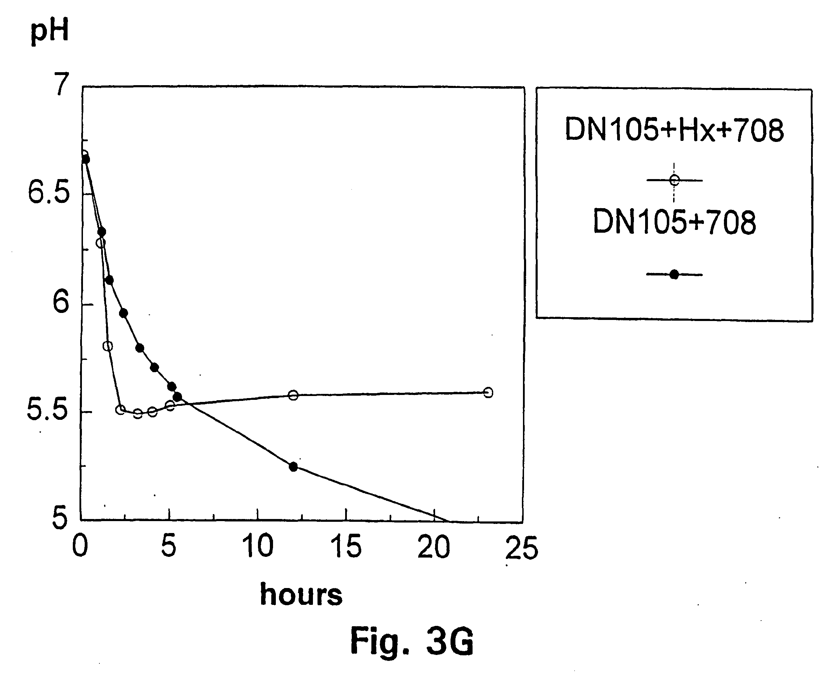

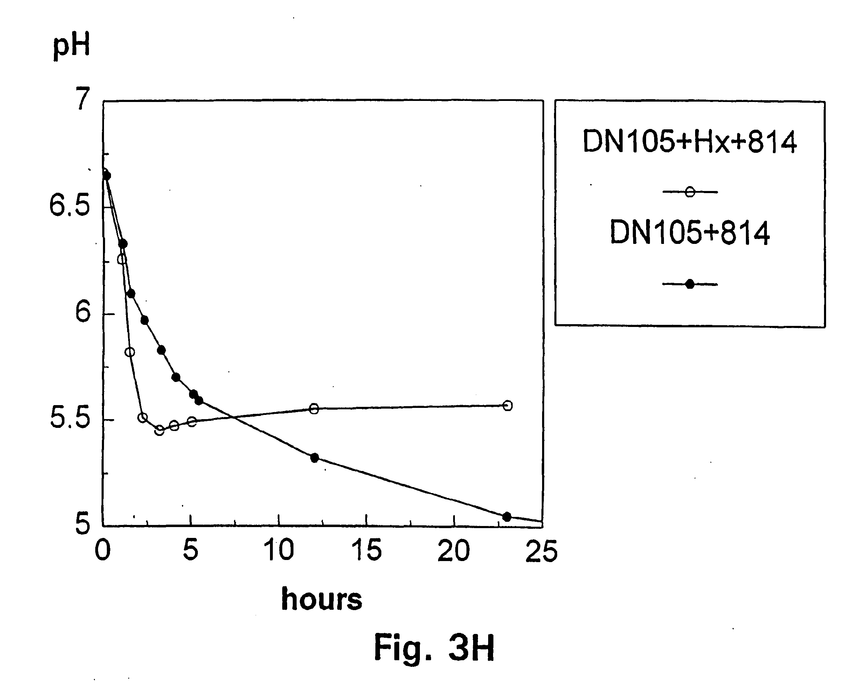

The addition of hypoxanthine was used as a positive control for the bacteriophage infection, as the addition of this purine compound enabled bacteriophage attack (Fig. 3 A-H). In all cultures without hypoxanthine added strain DN105 acidified the milk to around pH 5.0, whereas with the addition of hypoxanthine none of the cultures reached a pH of below 5.4.

### 1.4 Discussion

This Example shows that the starvation of a Pur strain of *L. lactis* for purines causes total resistance to a range of bacteriophages and that the Pur⁻ strain of *L. lactis* is capable of effectively acidifying milk in the presence of a large number of bacteriophages for which the corresponding wild type is susceptible.

Thus, the present Example shows that it is possible to develop strains which under appropriate selected conditions, where the strains are incapable of growth, are completely resistant to bacteriophages and that such strains have retained the metabolic ability to acidify milk. Such a system of bacteriophage resistance can be introduced into any bacteria e.g. *Lactococcus* spp., *Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Enterobacteriaceae* spp. including *E. coli, Corynebacterium* spp. and *Brevibacterium* spp. in regard to e.g. production of a bacterial fermentation product such as lactate, diacetyl, acetoin, methanethiol, ethanol etc. if proliferation of the bacteria is not a requirement.

### EXAMPLE 2

### The use of a thymidine auxotrophic Lactococcus lactis strain for obtaining resistance against bacteriophages in milk fermentations

### 2.1. Materials and methods

### (i) Bacterial strains, bacteriophages, media and growth conditions

The *Lactococcus lactis* strain MBP71 is a thymidine auxotrophic mutant (*thyA*) derived from the wild type strain CHCC373 (see Example 1). A sample of strain MBP71 has been deposited with Deutsche Sammlung von Mikroorganism und Zellkulturen GmbH, Mascheroder Weg 1b, D-38124 Braunschweig, Germany on 25 June 1999 under the accession No. DSM12891.

Strain CHCC373 was routinely grown at 30°C in M17 (Terzaghi & Sandine, 1975) supplemented with 0.5% of lactose, or in reconstituted skim milk (RSM) containing 9.5% (w/v) low fat skim milk powder. For the propagation of strain MBP71 the media were supplemented with 20 mg/I of thymidine, since neither M17 nor RSM support the growth of the ThyA phenotype. During the manipulations to construct strain MBP71 5 mg/l of erythromycin was added to the M17 based media to maintain the plasmid. Also, when growth at 37°C was required during these manipulations M17 based media were supplemented with 50 mM of NaCl to enhance growth. *E. coli* DH5α (Life Technologies Inc., USA) was grown in LB medium supplemented with 100 mg/I of erythromycin to maintain the transformed plasmid. The bacteriophage CHPC733 was used in growth experiments where strain MBP71 phage resistance was tested. For the bacteriophage handling and determination of titers see Example 1.

### (ii) Construction of plasmid with deletion in thyA from strain CHCC373

An about 800 bp fragment upstream of *thyA* from strain CHCC373 was obtained by PCR on chromosomal DNA using the primers
TATAATCTGCAGGGTCACACTATCAGTAATTG (SEQ ID NO:1) and
TATTTTAAGCTTCACAGTCTGCTATTTTGATTC (SEQ ID NO:2),
which furthermore introduce a *Pst*I and a *Hin*dIII site, respectively, in the fragment ends. The resulting fragment includes the -35 box of the *thyA* promoter, but not the - 10 box. Another about 800 bp fragment comprising an internal part of the *thyA* coding region was obtained by PCR on chromosomal DNA using the primers
TAAATTAAGCTTCGCAGACAAGATTTTTAAAC (SEQ ID NO:3) and
ATTTAAGTCGACGGCTCATAGTCCACAAGTTC (SEQ ID NO:4),
which introduce a *Hin*dIII and a *Sal*I site, respectively. The resulting fragment include the whole coding region of *ihyA* except the first 8 bp and the last 34 bp. The two PCR fragments were purified by using the QIAquick PCR purification kit (QIAGEN GmbH, Germany), cut with the indicated enzymes, and purified again. The pGhost9 vector (Maguin et al., 1996) was cut with the restriction enzymes *Pst*I and *Sal*I and purified. Thereafter, about 150 ng of the vector and about 50 ng of each of the two fragments were ligated overnight at 16°C in a total volume of 20µl. From this mixture 10 µl was used to transform *E. coli* DH5α. Plasmid DNA was isolated from possible clones by growing them overnight in LB medium with 100 mg/l of erythromycin and subsequently using 1.5 ml of these cultures with the QlAprep spin miniprep kit protocol (QIAGEN GmbH, Germany). The purified plasmid DNA was cut with *Pst*I and *Sal*I and one of them, pMBP63, yielded a band of approx. 1600 bp. This construct was further verified by PCR with the primers that were used to produce the two fragments, and also with the two outer primers. Finally, the construct was verified by sequencing both strands over the region of the deletion with the two primers
GACTGTTGCCCCATAGCG (SEQ ID NO:5) and
GCTTCGATTTTAGTATATGG (SEQ ID NO:6).

All primers were from TAG Copenhagen A/S, Copenhagen, Denmark.

### (iii) Inactivation of the chromosomal thyA gene in strain CHCC373

About 200 ng of pMBP63 was used to transform strain CHCC373. By employing the gene replacement feature (Biswas et al., 1993) of the pGhost9 vector the chromosomal *thyA* gene of strain CHCC373 was thereafter successfully inactivated. The resulting strain, MBP71, was shown to be a thymidine auxotrophic mutant.

### (iv) Test for acidification of RSM by strains CHCC373 and MBP71

Overnight cultures of M17 with 0.5% lactose and 20 mg/I of thymidine were washed twice in an isotonic solution of the same volume as the overnight culture to remove residual thymidine. The cells were resuspended in a fresh isotonic solution of a volume which was 1/20 of the overnight culture. These resuspended cells were used to inoculate RSM to a given volume identified by the percentage volume of the overnight culture i.e. an inoculation of 100% (v/v) indicates that 5 ml of the resuspended cells have been used to inoculate 100 ml of RSM. The pH was monitored either on-line or by measuring the pH of 2.5 ml samples.

### 2.2. Results of the acidification of RSM by Lactococcus lactis strain MBP71

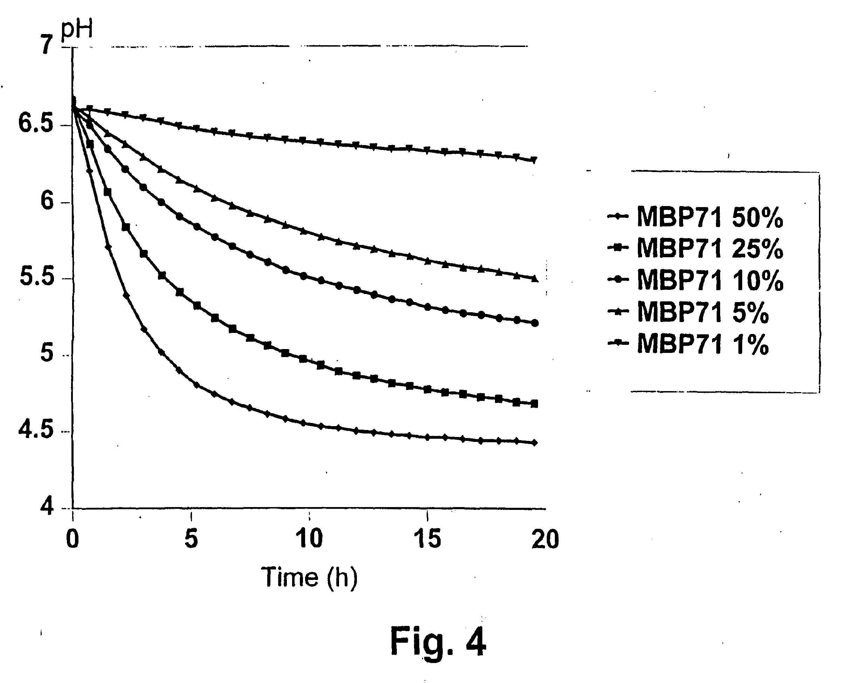

To test the acidification of RSM by strain MBP71 this strain was inoculated in varying amounts as described in materials and methods. The pH of the substrate material was then monitored on-line over a 20 hour period. The results shown in Fig. 4 clearly demonstrate that MBP71 is able to acidify milk down to at least pH 4.5 under thymidine starvation conditions. When thymidine was added to the RSM, strain MBP71 acidified the substrate material similarly to strain CHCC373 (details not shown).

### 2.3. Studies of the bacteriophage resistance of Lactococcus lactis MBP71 in RSM

As described above, a bacteriophage requires a susceptible host cell, which has intact DNA replication, RNA transcription and protein synthesis systems, for development. If a potential host is not capable of performing just one of these activities, bacteriophages can not proliferate in the cell. The thymidine auxotrophic strain MBP71 is only capable of DNA replication in the presence of thymidine, or another suitable precursor for dTTP. None of these precursors are present in RSM, and strain MBP71 is therefore not susceptible to bacteriophage attack under these conditions.

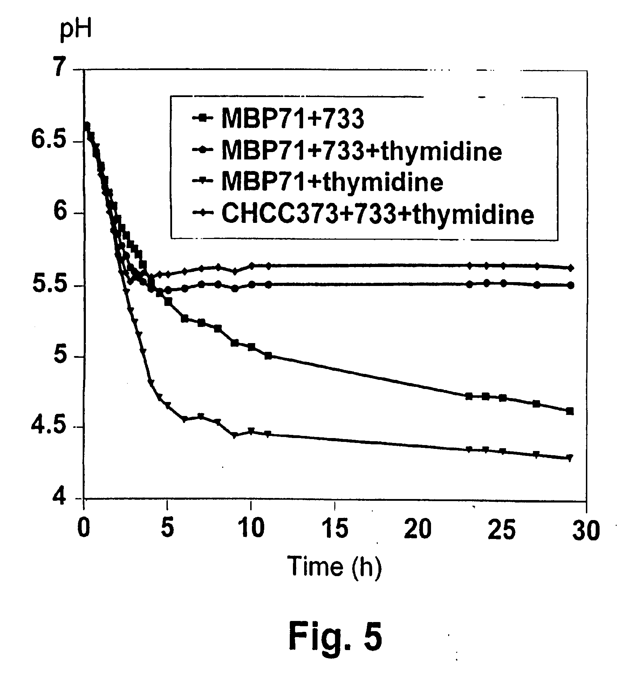

Fig. 5 shows the development of pH in 100 ml RSM inoculated with strain MBP71 at a concentration of 25% (v/v). When thymidine was present it was added to a final concentration of 20 mg/l. When the bacteriophage CHPC733 was present it was added to a concentration of 2x10⁸ PFU/ml half an hour after cell inoculation. This gives a MOI (multiplicity of infection) of 0.2. The results in Fig. 5 clearly demonstrate that strain MBP71 acidifies RSM without thymidine down to at least pH 4.6 even in the presence of bacteriophages. However, when thymidine is added to the RSM strain MBP71 becomes susceptible to bacteriophage attack and the milk is only acidified down to pH 5.5, which is also close to the pH that is reached for the wild type strain CHCC373 in the presence of bacteriophages. If the bacteriophages are omitted, but thymidine is added, strain MBP71 acidifies the RSM similarly to strain CHCC373 (see Fig. 5, details not shown).

### 2.4. Discussion

This example shows that the starvation of a *thyA* strain of *L. lactis* for thymidine causes total resistance to bacteriophage CHPC733 and that the *ihyA* strain of *L. lactis* is capable of effectively acidifying milk under these conditions. This is contrary to the wild type, which is not able to acidify the milk to an acceptable level under the same conditions. Although, strain MBP71 has only been tested for bacteriophage resistance to one bacteriophage it is believed that strain MBP71 is resistant to all, or most, types of bacteriophages.

### REFERENCES

1. Biswas, I., A. Gruss, S.D. Ehrlich and E. Maguin. 1993. High-efficiency gene inactivation and replacement system for gram-positive bacteria. J. Bacteriol. 175:3628-3635.
2. Dickely, F., D. Nilsson, E. B. Hansen and E. Johansen. 1995. Isolation of Lactococcus lactis nonsense suppressors and construction of a food-grade cloning vector. Mol. Microbiol. 15:839-847.
3. Maguin, E., H. Prévost, S.D. Ehrlich and A. Gruss. 1996. Efficient insertional mutagenesis in Lactococci and other gram-positive bacteria. J. Bacteriol. 178:931-935.
4. Nilsson, D., and A. A. Lauridsen. 1992. Isolation of purine auxothrophic mutants of Lactococcus lactis and characterisation of the gene hpt encoding hypoxanthine guanine phosphoribosyltransferase. Mol. Gen. Genet. 235:359-364.
5. Richardson, G.H., C.A. Ernstrom, J.M. Kim and C. Daly. 1983. Proteinase negative variants of Streptococcus cremoris for cheese starters. J. Dairy Sci. 66:2278-2286.
6. Richardson, G.H., A.Y. Gamay, M.A. Shelaih, J.M. Kim and C.L. Hansen. 1984. Paired and single strain protease negative lactic Streptococci for cheese manufacturing. J. Dairy Sci. 67:518-521.
7. Terzaghi, B. E., and W. E. Sandine. 1975. Improved medium for lactic streptococci and their bacteriophages. Appl. Microbiol. 29:807-813.

## Claims

1. A method of modifying a substrate material by means of a bacterial culture which is capable of being metabolically active in said substrate, whereby the bacterial culture is not susceptible to attack by bacteriophages, the method comprising
(i) isolating a bacterial strain which is not capable of DNA replication in said substrate material but is capable of metabolically modifying the substrate material, wherein the bacterial strain is a mutant strain being auxotrophic in respect of a compound which is not present in the substrate material and which is required by the strain for DNA replication,
(ii) propagating the selected strain in a medium wherein the strain is capable of replicating to obtain a culture of said strain,
(iii) adding the thus obtained bacterial culture to the substrate material and keeping the material under conditions where the culture is metabolically active,
whereby, if the substrate material is contaminated with a bacteriophage, the metabolic activity of the bacterial culture is substantially unaffected by the bacteriophage, and
wherein the bacterial culture is selected from the group consisting of *Lactococcus* spp., *Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Corynebacterium* spp. and *Brevibacterium* spp; and
wherein the substrate material is a starting material for an edible product, the material is selected from the group consisting of milk, a vegetable material, a meat product, a must, a fruit juice, a wine, a dough and a batter; and
wherein the bacterial culture as obtained in the method is used as a starter culture in the preparation of a product selected from the group consisting of a dairy flavour, a product for cheese flavouring, a food product and a feed product.

2. The method according to claim 1, wherein the mutant strain is a Pur mutant including *Lactococcus lactis* strain DN105 deposited under the accession number DSM 12289.

3. The method according to claim 1, wherein the mutant strain is a *thyA* mutant including *Lactococcus lactis* strain MBP71 deposited under the accession number DSM12891.

4. The method according to any of claims 1 to 3, wherein the bacterial culture is of *Lactococcus lactis*.

5. The method according to any of claims 1 to 4, wherein the bacterial strain is added to the substrate material at a concentration in the range of 10⁵ to 10⁹ CFU/ml or g of the material.

6. The method according to any of claims 1 to 5, wherein the culture comprises a genetically modified strain which, relative to its parent strain is enhanced in at least one metabolic pathway.

7. The method according to claim 6, wherein the genetically modified strain has, relative to its parent strain, an enhanced metabolic activity selected from the group consisting of enhanced glycolytic flux and enhanced flux through the pentose phosphate pathway.

8. The method according to claim 7, wherein the genetically modified strain has, relative to its parent strain, an enhanced ATPase activity.

9. The method according to claim 1, wherein the bacterial culture comprises a strain which is a conditional mutant which at a predetermined condition does not perform DNA replication.

10. A method according to claim 9, wherein the predetermined condition is selected from the group consisting of pH, temperature, composition of the substrate material and presence/absence of an inducer substance.

11. The method according to claim 1, wherein the culture comprises a bacterial strain which is capable of increasing the size of the cells without mitosis.

12. Use of a culture as obtained in the method of any of claims 1 to 11 as a starter culture in the preparation of a product selected from the group consisting of a dairy flavour, a product for cheese flavouring, a food product and a feed product.

13. A mutant lactic acid bacterium, wherein the mutant strain is the *thyA* mutant *Lactococcus lactis* strain MBP71 deposited under the accession number DSM12891.

## Patentansprüche

1. Ein Verfahren zur Modifizierung eines Substratmaterials mit Hilfe einer Bakterienkultur, die dazu in der Lage ist, im genannten Substrat metabolisch aktiv zu sein, wobei die Bakterienkultur nicht für einen Angriff durch Bakteriophagen empfänglich ist; dieses Verfahren umfasst
(i) die Isolierung eines Bakterienstamms, der nicht zu einer DNA-Replikation im genannten Substratmaterial, jedoch zu einer metabolischen Modifizierung des Substratmaterials in der Lage ist, wobei der Bakterienstamm ein Mutantenstamm ist, der auxotroph in Bezug auf eine Verbindung ist, die nicht im Substratmaterial vorhanden ist und die vom Stamm zur DNA-Replikation benötigt wird,
(ii) die Vermehrung des ausgewählten Stamms in einem Medium, in dem der Stamm zur Replikation in der Lage ist, um eine Kultur des genannten Stamms zu erzielen,
(iii) die Hinzufügung der so erzielten Bakterienkultur zum Substratmaterial und das Aufbewahren des Materials unter Bedingungen, unter denen die Kultur metabolisch aktiv ist,
wobei, falls das Substratmaterial von einer Bakteriophage kontaminiert wird, die metabolische Aktivität der Bakterienkultur im Wesentlichen von der Bakteriophage nicht beeinflusst wird, und
wobei die Bakterienkultur aus der Gruppe ausgewählt wird, die aus *Lactococcus* spp., *Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Corynebacterium* spp. und *Brevibacterium* spp besteht, und
wobei das Substratmaterial ein Ausgangsmaterial für ein essbares Produkt ist; das Material wird aus einer Gruppe ausgewählt, die aus Milch, einem pflanzlichen Material, einem Fleischprodukt, einem Most, einem Fruchtsaft, einem Wein, einem Teig und einem Backteig besteht; und
wobei die Bakterienkultur, die über dieses Verfahren erzielt wurde, als Ausgangsmaterial bei der Zubereitung eines Produkts verwendet wird, das aus einer Gruppe ausgewählt wird, die ein Molkereiaroma, ein Produkt zur Käsearomatisierung, ein Nahrungsmittelprodukt und ein Futtermittelprodukt umfasst.

2. Das Verfahren nach Anspruch 1, wobei der Mutantenstamm eine Pur-Mutante einschließlich eines Stamms DN105 des *Lactococcus lactis* mit der Hinterlegungsnummer DSM 12289 ist.

3. Das Verfahren nach Anspruch 1, wobei der Mutantenstamm eine thyA-Mutante einschließlich eines Stamms MBP71 des *Lactococcus lactis* mit der Hinterlegungsnummer DSM 12891 ist.

4. Das Verfahren nach den Ansprüchen 1 bis 3, wobei die Bakterienkultur aus *Lactococcus lactis* besteht.

5. Das Verfahren nach den Ansprüchen 1 bis 4, wobei der Bakterienstamm dem Substratmaterial in einer Konzentration im Bereich von 10⁵ bis 10⁹ KbE/ml oder g des Materials hinzugefügt wird.

6. Das Verfahren nach den Ansprüchen 1 bis 5, wobei die Kultur einen genetisch modifizierten Stamm umfasst, der im Vergleich mit dem Mutterstamm bei mindestens einem metabolischen Weg verbessert ist.

7. Das Verfahren nach dem Anspruch 6, wobei der genetisch modifizierte Stamm im Vergleich zu dem Mutterstamm eine verbesserte metabolische Aktivität hat, welche aus einer Gruppe ausgewählt wird, die aus einem verbesserten glykolytischen Fluss und einem verbesserten Fluss durch den Pentosephosphatweg besteht.

8. Das Verfahren nach dem Anspruch 7, wobei der genetisch modifizierte Stamm eine im Vergleich zum Mutterstamm verbesserte ATPase-Aktivität hat.

9. Das Verfahren nach dem Anspruch 1, wobei die Bakterienkultur einen Stamm umfasst, welcher eine konditionelle Mutante ist, bei der sich in einer vorgegebenen Bedingung keine DNA-Replikation vollzieht.

10. Ein Verfahren nach dem Anspruch 9, wobei die vorgegebene Bedingung aus einer Gruppe ausgewählt wird, die aus dem pH-Wert, der Temperatur, der Zusammensetzung des Substratmaterials und dem Vorhandensein/dem Fehlen einer Induktorsubstanz besteht.

11. Das Verfahren nach dem Anspruch 1, wobei die Kultur einen Bakterienstamm umfasst, der in der Lage ist, die Größe der Zellen ohne Mitose zu steigern.

12. Verwendung einer Bakterienkultur, die über das Verfahren eines der Ansprüche 1 bis 11 erzielt wurde, als Ausgangsmaterial bei der Zubereitung eines Produkts, das aus einer Gruppe ausgewählt wird, die ein Molkereiaroma, ein Produkt zur Käsearomatisierung, ein Nahrungsmittelprodukt und ein Futtermittelprodukt umfasst.

13. Ein mutierendes Milchsäurebakterium, wobei der Mutantenstamm die *thyA-*Mutante des Stamms MBP71 des *Lactococcus lactis* mit der Hinterlegungsnummer DSM 12891 ist.

## Revendications

1. Une méthode de modification d'un substrat au moyen d'une culture bactérienne capable d'une activité métabolique dans le support qui la rend non susceptible d'attaque par les bactériophages, laquelle méthode comprend :
(i) l'isolation d'une souche bactérienne incapable de réplication d'ADN dans ledit substrat mais capable de modifier celui-ci métaboliquement, **caractérisée en ce qu'**elle forme une souche mutante auxotrophique par rapport à un composé qui n'est pas présent dans le substrat et dont la souche a besoin pour la réplication d'ADN,
(ii) la propagation de la souche sélectionnée dans un milieu dans lequel elle est capable de se reproduire pour donner une culture,
(iii) l'incorporation dans le substrat de la culture bactérienne ainsi obtenue et la conservation de cette matière dans des conditions d'activité métabolique de la culture
**caractérisée en ce qu'**une contamination du substrat par un bactériophage n'affecte pas de manière appréciable l'activité métabolique de la culture bactérienne ;
**caractérisée en ce que** la culture bactérienne est choisie dans le groupe composé de *Lactococcus* spp., *Lactobacillus* spp., *Leuconostoc* spp., *Pediococcus* spp., *Streptococcus* spp., *Propionibacterium* spp., *Bifidobacterium* spp., *Staphylococcus* spp., *Micrococcus* spp., *Bacillus* spp., *Actinomycetes* spp., *Corynebacterium* spp. et *Brevibacterium* spp ;
**caractérisée en ce que** le substrat, composant initial d'un produit comestible, est sélectionné dans le groupe comprenant le lait, une matière végétale, un produit carné, un moût, un jus de fruit, un vin, une pâte à pain et une pâte à frire, et
**caractérisée en ce que** la culture bactérienne obtenue selon cette méthode est utilisée comme starter dans la préparation d'un produit choisi dans le groupe comprenant un arôme laitier, un produit d'aromatisation de fromages, un produit d'alimentation humaine et un produit d'alimentation animale.

2. La méthode selon la revendication 1, **caractérisée en ce que** la souche mutante est un mutant pur incluant une souche de *Lactococcus lactis* DN105 déposée sous le code d'accession DSM 12289.

3. La méthode selon la revendication 1, **caractérisée en ce que** la souche mutante est un mutant *thyA* incluant une souche de *Lactococcus lactis* MBP71 déposée sous le code d'accession DSM 12891.

4. La méthode selon l'une des revendications 1 à 3, **caractérisée en ce que** la culture bactérienne est une culture de *Lactococcus lactis.*

5. La méthode selon l'une des revendications 1 à 4, **caractérisée en ce que** la souche bactérienne est incorporée dans le substrat dans une concentration de l'ordre de 10⁵ à 10⁹ CFU/ml ou g du substrat.

6. La méthode selon l'une des revendications 1 à 5, **caractérisée en ce que** la culture comprend une souche génétiquement modifiée renforcée par rapport à la souche parente sur au moins une des voies métaboliques.

7. La méthode selon la revendication 6, **caractérisée en ce que** la souche génétiquement modifiée présente, par rapport à sa souche parente, une activité métabolique renforcée choisie dans le groupe qui consiste en un flux glycolytique renforcé et en un flux renforcé par la voie du pentose phosphate.

8. La méthode selon la revendication 7, **caractérisée en ce que** la souche génétiquement modifiée présente, par rapport à sa souche parente, une activité ATPasique renforcée.

9. La méthode selon la revendication 1, **caractérisée en ce que** la culture bactérienne contient un mutant conditionnel qui, dans certaine condition prédéterminée, ne réalise pas de réplication d'ADN.

10. Une méthode selon la revendication 9, **caractérisée en ce que** la condition prédéterminée est choisie dans le groupe dont font partie l'indice pH, la température, la composition du matériau substrat et la présence ou absence d'une substance inductive.

11. La méthode selon la revendication 1, **caractérisée en ce que** la culture bactérienne comprend une souche bactérienne capable d'augmenter sans mitose la taille des cellules.

12. l'utilisation d'une culture bactérienne, obtenue selon la méthode conforme aux revendications 1 à 11, comme starter dans la préparation d'un produit choisi dans le groupe composé d'un arôme laitier, d'un produit d'aromatisation de fromages, d'un produit d'alimentation humaine et d'un produit d'alimentation animale.

13. Une bactérie mutante de l'acide lactique, **caractérisée en ce que** la souche mutante est la souche *à mutant thyA* de *Lactococcus lactis* MBP71, déposée sous le code d'accession DSM 12891.


### SEQUENCE LISTING

<110> Dan Nilsson
   Thomas Janzen
<120> Method of preventing bacteriophage infection of bacterial cultures
<130> 21134 PC 1
<150> PA 1998 00878
   <151> 1998-07-03
<150> US 60/091,735
   <151> 1998-07-06
<160> 6
<170> FastSEQ for Windows Version 3.0
<210> 1
   <211> 32
   <212> DNA
   <213> Artificial Sequence
<220>
   <223> PCR-primer for the construction of plasmid with deletion in thyA from strain CHCC373
<400> 1
   tataatctgc agggtcacac tatcagtaat tg 32
<210> 2
   <211> 33
   <212> DNA
   <213> Artificial Sequence
<220>
   <223> PCR-primer for the construction of plasmid with deletion in thyA from strain CHCC373
<400> 2
   tattttaagc ttcacagtct gctattttga ttc 33
<210> 3
   <211> 32
   <212> DNA
   <213> Artificial Sequence
<220>
   <223> PCR-primer for the construction of plasmid with deletion in thyA from strain CHCC373
<400> 3
   taaattaagc ttcgcagaca agatttttaa ac 32
<210> 4
   <211> 32
   <212> DNA
   <213> Artificial Sequence
<220>
   <223> PCR-primer for the construction of plasmid with deletion in thyA from strain CHCC373
<400> 4
   atttaagtcg acggctcata gtccacaagt tc 32
<210> 5
   <211> 18
   <212> DNA
   <213> Artificial Sequence
<220>
   <223> Sequencing primer used for the verification of the construction of plasmid with deletion in thyA from strain CHCC373.
<400> 5
   gactgttgcc ccatagcg 18
<210> 6
   <211> 20
   <212> DNA
   <213> Artificial Sequence
<220>
   <223> Sequencing primer used for the verification of the construction of plasmid with deletion in thyA from strain CHCC373.
<400> 6
   gcttcgattt tagtatatgg 20